# EUROPEAN PATENT SPECIFICATION

(11) **EP 3 344 018 B1**
(45) Date of publication and mention of the grant of the patent: **09.12.2020**
(21) Application number: 15902901.6
(22) Date of filing: 28.08.2015
(51) Int. Cl.: H05H 1/24, H01J 37/32, A61L 2/14

(54) **PLASMA IRRADIATION DEVICE**
PLASMAGASBESTRAHLUNGSVORRICHTUNG
DISPOSITIF D'IRRADIATION PLASMA

(43) Date of publication of application: 04.07.2018
(73) Proprietor: FUJI Corporation, Chiryu Aichi (JP)
(72) Inventor: IKEDO, Toshiyuki, Chiryu Aichi (JP); JINDO, Takahiro, Chiryu Aichi (JP)
(74) Representative: Grünecker Patent- und Rechtsanwälte PartG mbB
(86) International application number: PCT/JP2015/074382
(87) International publication number: WO 2017/037775

(56) References cited:
- JP-A- H09 213 497
- JP-A- 2002 151 494
- JP-A- 2005 174 879
- JP-A- 2005 174 879
- JP-A- 2008 187 062
- JP-A- 2011 207 736

## Description

### Technical Field

The present invention relates to a plasma irradiation apparatus which irradiates a processing target object with a plasma.

### Background Art

The plasma irradiation apparatus irradiates the processing target object with a plasma by ejecting the plasma from an ejection port. At this time, fixing the irradiation conditions of the plasma is desirable and technology for adjusting the distance between the processing target object and the ejection port of the plasma is described in PTL 1.

JP 2002 151494 A and JP 2005 174879 A both relate to plasma processing apparatus controlling inert gas flow.

PTL 1: JP-A-2014-113534

### Summary of Invention

### Technical Problem

According to the technology which is described in PTL 1, of the irradiation condition of the plasma, it is possible to adjust the irradiation distance. However, since the plasma contains active radicals, when the active radicals react with oxygen, the active radicals become ozone and the effect of the plasma irradiation is reduced. Therefore, it is preferable to manage the concentration conditions of a specific gas such as oxygen in the periphery of the processing target object during the plasma irradiation as a plasma irradiation condition. The present invention is made in consideration of such issues and an object of the present invention is to manage the concentration conditions of a specific gas in the periphery of the processing target object.

### Solution to Problem

In order to solve the problem, a plasma irradiation apparatus according to claim 1 is provided.

### Advantageous Effects of Invention

In the plasma irradiation apparatus of the present specification, after a predetermined gas such as an inert gas is supplied to the inner portion of the cover housing, the plasma is ejected toward the inner portion of the cover housing. Even during the ejection of the plasma, the supplying of the inert gas to the inner portion of the cover housing is continued. Accordingly, it becomes possible to vent the specific gas such as air using the inert gas from the inner portion of the cover housing and it becomes possible to manage the concentration conditions of the specific gas in a periphery of a processing target object.

### Brief Description of Drawings

[Fig. 1] Fig. 1 is a perspective view of an atmospheric pressure plasma irradiation apparatus.
[Fig. 2] Fig. 2 is an exploded diagram of a plasma generation device.
[Fig. 3] Fig. 3 is an exploded diagram of the plasma generation device.
[Fig. 4] Fig. 4 is a sectional view of the plasma generation device.
[Fig. 5] Fig. 5 is a perspective view of the atmospheric pressure plasma irradiation apparatus.
[Fig. 6] Fig. 6 is a side view of the atmospheric pressure plasma irradiation apparatus.
[Fig. 7] Fig. 7 is a side view of the atmospheric pressure plasma irradiation apparatus.
[Fig. 8] Fig. 8 is a perspective view of the atmospheric pressure plasma irradiation apparatus.
[Fig. 9] Fig. 9 is a schematic diagram of a cooling device.
[Fig. 10] Fig. 10 is a block diagram of a control device.

### Description of Embodiments

Hereinafter, a detailed description will be given of an example of the present invention with reference to the drawings as an embodiment of the present invention.

### <Configuration of Atmospheric Pressure Plasma Irradiation Device>

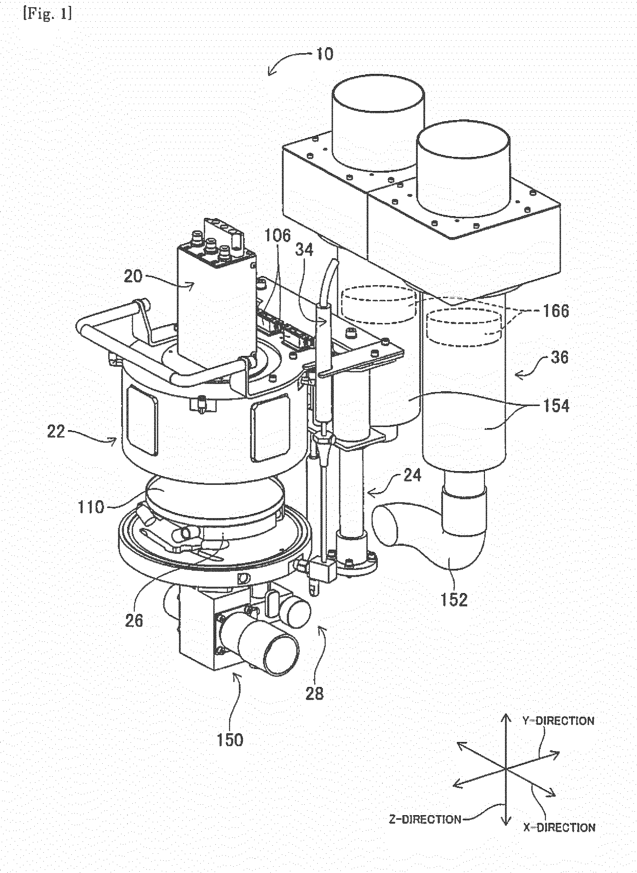

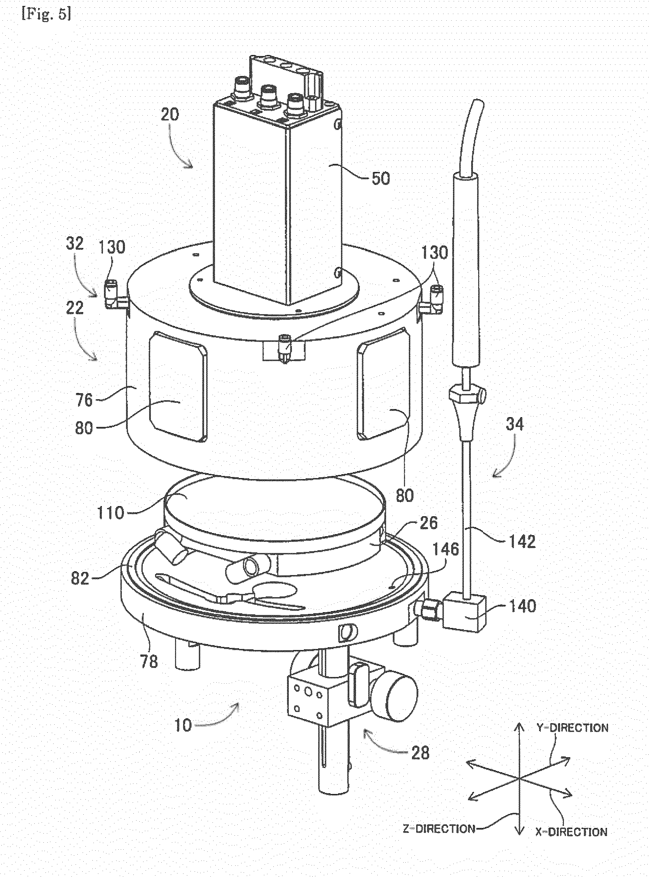

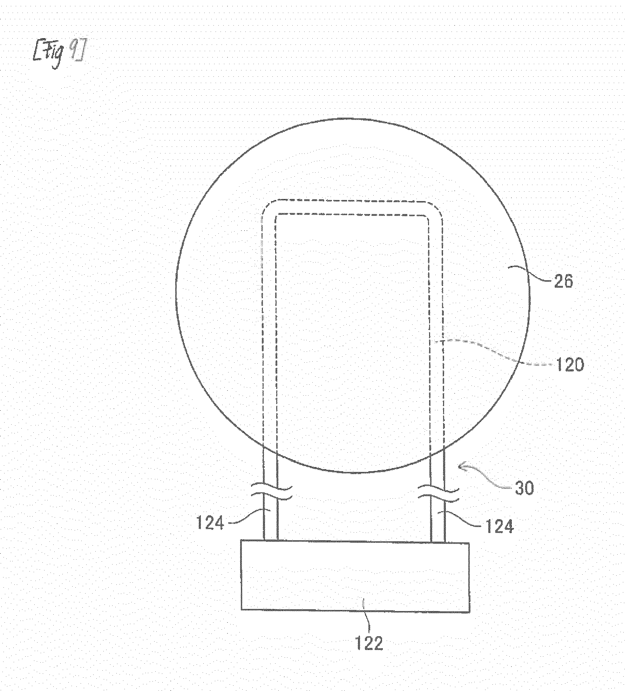

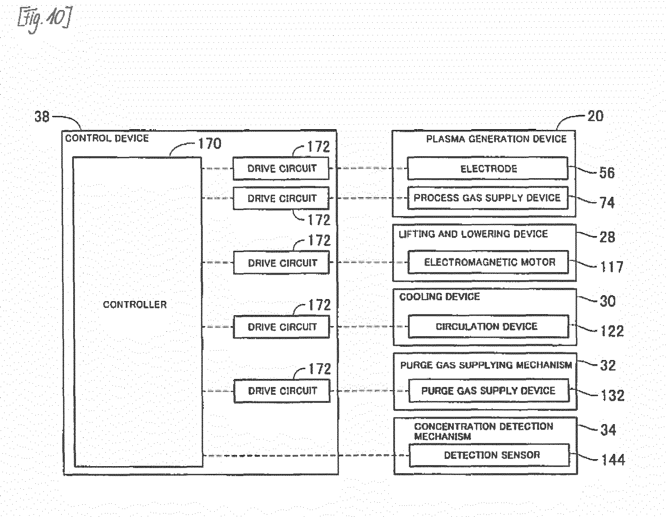

Fig. 1 illustrates an atmospheric pressure plasma irradiation apparatus 10 of the example of the present invention. The atmospheric pressure plasma irradiation apparatus 10 is an apparatus for irradiating a culture medium with a plasma under atmospheric pressure and is provided with a plasma generation device 20, a cover housing 22, an open-close mechanism 24, a stage 26, a lifting and lowering device 28, a cooling device (refer to Fig. 9) 30, a purge gas supplying mechanism (refer to Fig. 5) 32, a concentration detection mechanism 34, a ventilation mechanism 36, and a control device (refer to Fig. 10) 38. A width direction of the atmospheric pressure plasma irradiation apparatus 10 is referred to as an X-direction, a depth direction of the atmospheric pressure plasma irradiation apparatus 10 is referred to as a Y-direction, and a direction which is perpendicular to the X-direction and the Y-direction, that is, the up-down direction is referred to as a Z-direction.

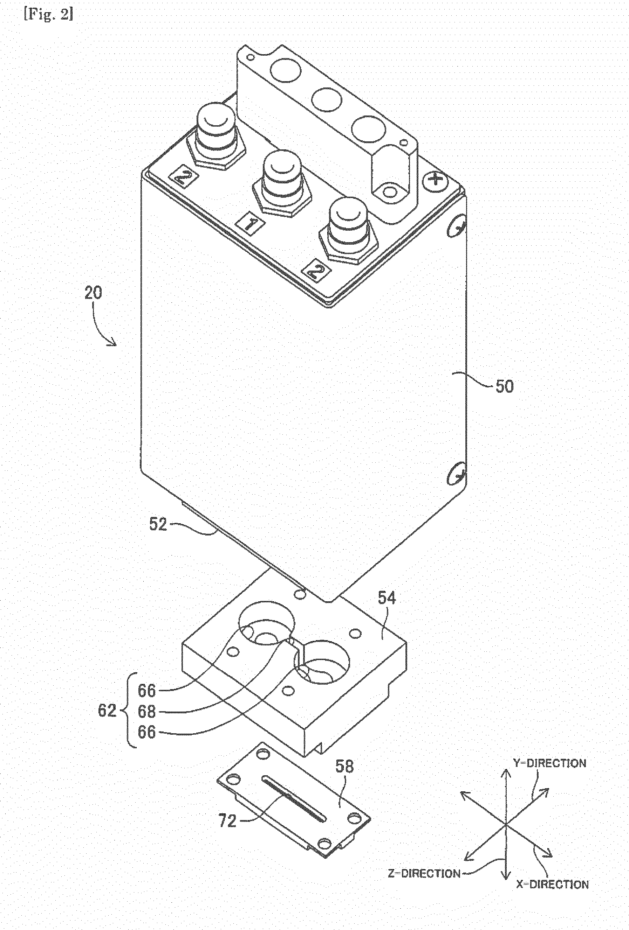

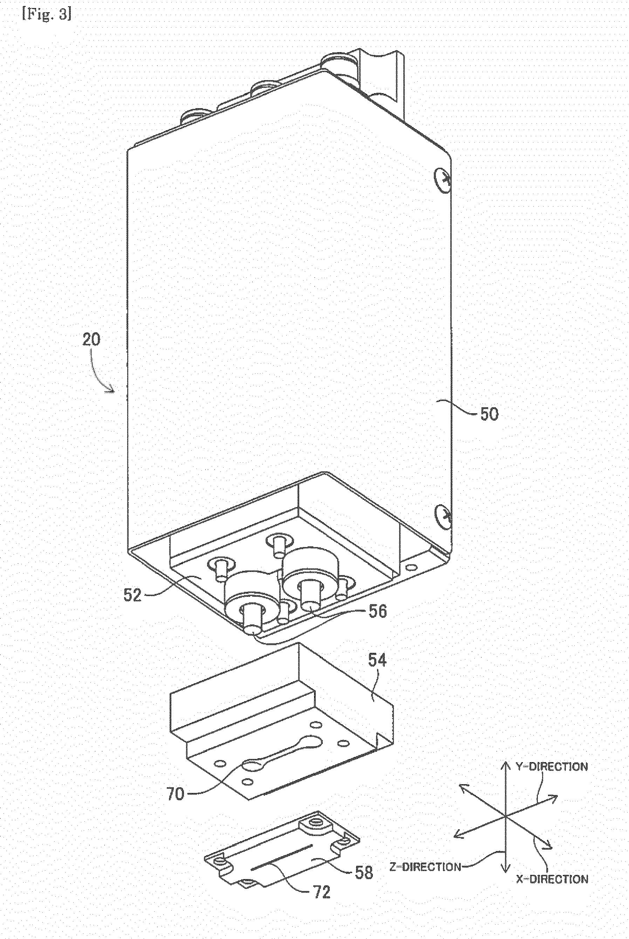

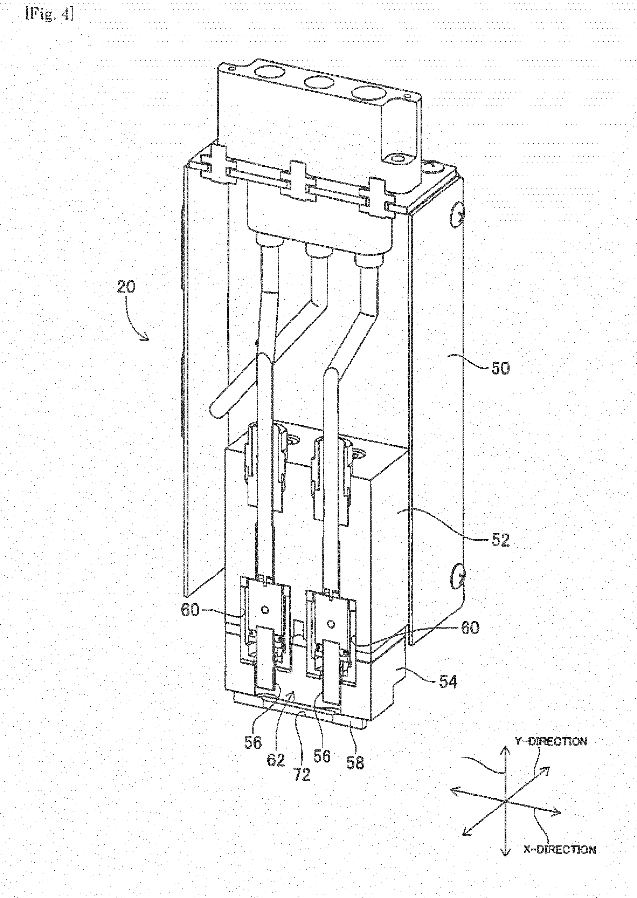

As illustrated in Figs. 2 to 4, the plasma generation device 20 includes a cover 50, an upper block 52, a lower block 54, a pair of electrodes 56, and a nozzle block 58. The cover 50 has a substantially lidded quadrilateral tubular shape and the upper block 52 is installed in the inner portion of the cover 50. The upper block 52 has a substantially parallelepiped shape and is molded using a ceramic. A pair of column recessed sections 60 having a columnar shape are formed on the lower face of the upper block 52.

The lower block 54 also has a substantially parallelepiped shape and is molded using a ceramic. A recessed section 62 is formed on the upper face of the lower block 54 and the recessed section 62 is constituted by a pair of column recessed sections 66 having a columnar shape and a connecting recessed section 68 which connects the pair of columnar recessed sections 66. In a state in which the lower block 54 protrudes from the lower end of the cover 50, the lower block 54 is fixed to the lower face of the upper block 52 and the column recessed sections 60 of the upper block 52 are connected to the column recessed sections 66 of the lower block 54. The column recessed sections 60 and the column recessed sections 66 have approximately the same diameters. A slit 70 which passes through to the lower face of the lower block 54 is formed in the bottom face of the recessed section 62.

Each of the electrodes 56 of the pair is installed in a columnar cavity which is formed through partitioning by the column recessed section 60 of the upper block 52 and the column recessed section 66 of the lower block 54. The outer diameters of the electrodes 56 are smaller than the inner diameters of the column recessed sections 60 and 66. The nozzle block 58 is substantially plate-shaped and is fixed to the lower face of the lower block 54. An ejection port 72 which communicates with the slit 70 of the lower block 54 is formed in the nozzle block 58 and the ejection port 72 penetrates the nozzle block 58 in the up-down direction.

The plasma generation device 20 further includes a process gas supply device (refer to Fig. 10) 74. The process gas supply device 74 is a device which supplies a process gas which is a combination of an active gas such as oxygen and an inert gas such as nitrogen at an arbitrary ratio and is connected to columnar cavities which are formed through partitioning by the column recessed sections 60 and 66 and the top portion of the connecting recessed section 68 via a pipe (not illustrated). Accordingly, the process gas is supplied to the inner portion of the recessed section 62 from the gaps between the electrodes 56 and the column recessed sections 66 and the top portion of the connecting recessed section 68.

According to this structure, the plasma generation device 20 ejects a plasma from the ejection port 72 of the nozzle block 58. In detail, the process gas is supplied to the inner portion of the recessed section 62 by the process gas supply device 74. At this time, a voltage is applied to the pair of electrodes 56 in the recessed section 62 and a current flows between the pair of electrodes 56. Accordingly, discharging occurs between the pair of electrodes 56 and the process gas is turned into a plasma by the discharging. The plasma is ejected from the ejection port 72 via the slit 70.

As illustrated in Fig. 5, the cover housing 22 includes an upper cover 76 and a lower cover 78. The upper cover 76 has a substantially lidded cylindrical shape and a through hole (not illustrated) of a shape corresponding to the lower block 54 of the plasma generation device 20 is formed in a lid portion of the upper cover 76. The cover 50 of the plasma generation device 20 is fixed to cover the through hole in a state of being provided to stand on the lid portion of the upper cover 76. Therefore, the lower block 54 of the plasma generation device 20 and the nozzle block 58 protrude toward the inner portion of the upper cover 76 to extend in the Z-direction. Accordingly, the plasma which is generated by the plasma generation device 20 is ejected in the Z-direction from the ejection port 72 of the nozzle block 58 toward the inner portion of the upper cover 76.

Rectangular through holes (not illustrated) are formed in three equidistant positions in the side face of the upper cover 76 and a transparent glass plate 80 is installed to block the through holes. Accordingly, it is possible to visually recognize the inner portion of the upper cover 76 via the glass plate 80.

The lower cover 78 of the cover housing 22 is substantially disc shaped and is fixed to the housing (not illustrated) of a placement section on which the atmospheric pressure plasma irradiation apparatus 10 is mounted. The outer diameter of the lower cover 78 is greater than the outer diameter of the upper cover 76 and a ring-shaped packing 82 of the same diameter as the upper cover 76 is installed on the upper face of the lower cover 78. Due to the upper cover 76 being caused to slide downward by the open-close mechanism 24, the upper cover 76 adheres to the packing 82 and a state is attained in which the inner portion of the cover housing 22 is sealed.

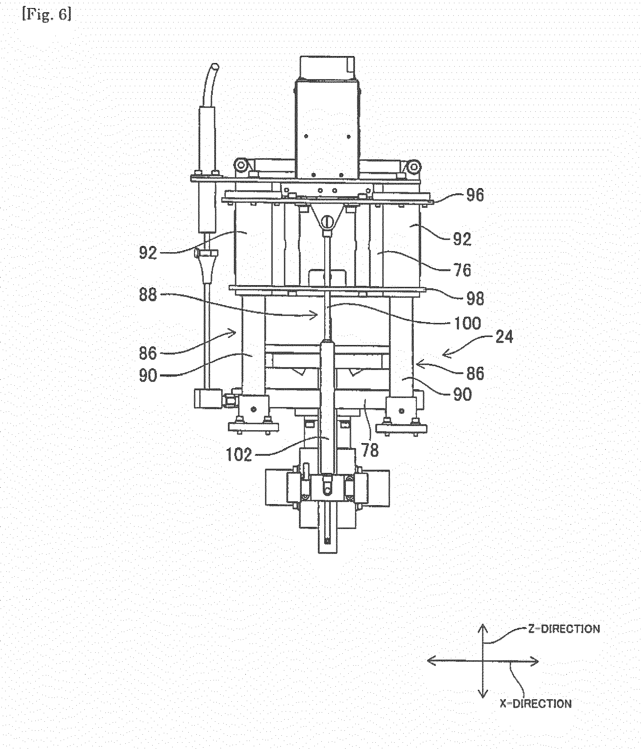

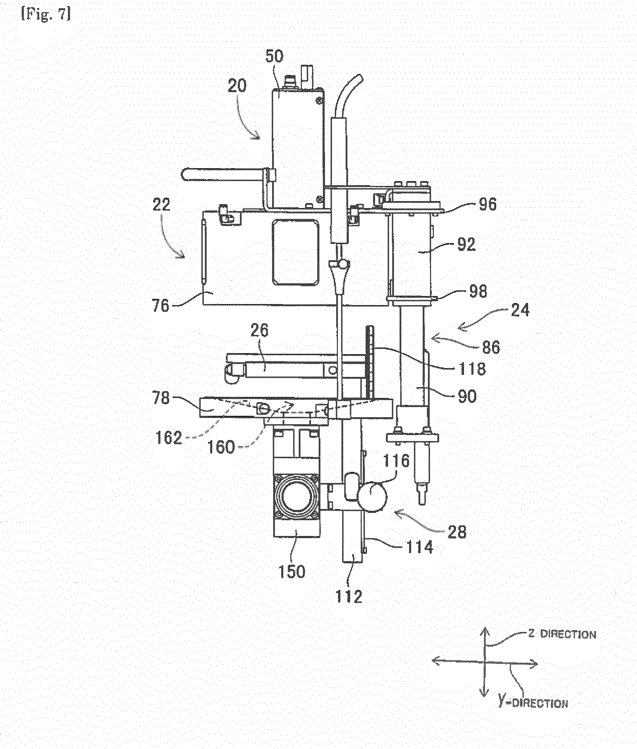

In detail, as illustrated in Figs. 6 and 7, the open-close mechanism 24 includes a pair of slide mechanisms 86 and an air cylinder 88. Each of the slide mechanisms 86 includes a support shaft 90 and a slider 92. The support shafts 90 are provided to stand on the housing of the placement section so as to extend in the Z-direction. The sliders 92 have substantially cylindrical shapes and are fitted onto the outside of the support shafts 90 to be slidable in the axial directions of the support shafts 90. The upper cover 76 is held onto the sliders 92 by an upper bracket 96 and a lower bracket 98. Accordingly, the upper cover 76 is slidable in the Z-direction, that is, the up-down direction.

The air cylinder 88 includes a rod 100, a piston (not illustrated), and a cylinder 102. The rod 100 is installed to extend in the Z-direction and is fixed to the upper cover 76 on the upper end portion. The piston is fixed to the lower end portion of the rod 100. The piston fits into the inner portion of the cylinder 102 from the upper end of the cylinder 102 and moves to be capable of sliding in the inner portion of the cylinder 102. The cylinder 102 is fixed to the housing of the placement section by the lower end portion and a predetermined amount of air is sealed into the inner portion of the cylinder 102.

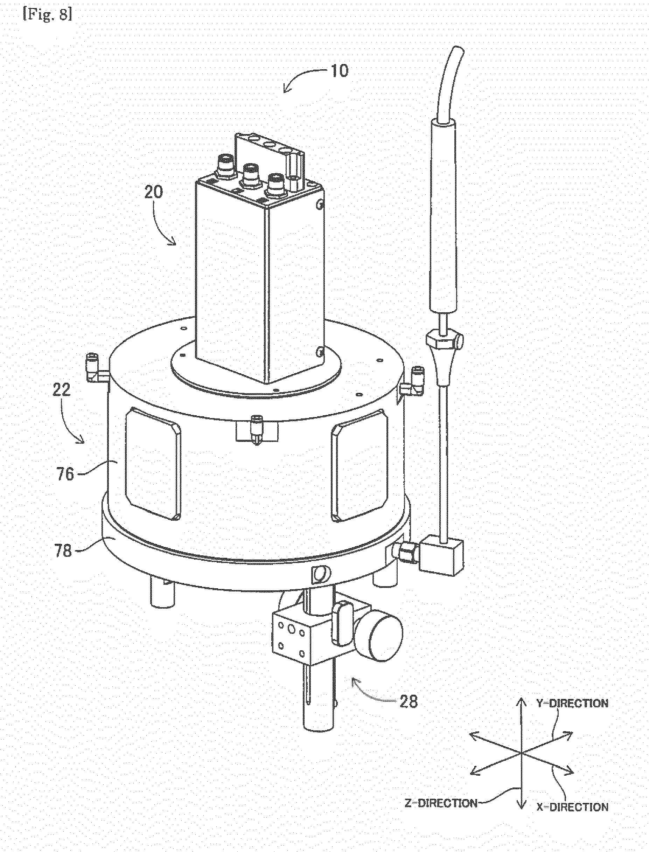

Accordingly, the air cylinder 88 functions as a damper and sudden falling of the upper cover 76 is prevented. The air pressure of the inner portion of the cylinder 102 is a pressure that can be compressed by the weight of an integrated object which slides with the upper cover 76, that is, the weight of the upper cover 76, the plasma generation device 20, the slider 92, and the like. In other words, when an operator releases the upper cover 76 in a state in which the upper cover 76 is lifted, the upper cover 76 is lowered by the weight of the upper cover 76 itself and the like. The upper cover 76 adheres to the packing 82 of the lower cover 78, and as illustrated in Fig. 8, a state is attained in which the inner portion of the cover housing 22 is sealed by the upper cover 76 and the lower cover 78.

The inner portion of the cover housing 22 is exposed by the operator lifting the upper cover 76. A magnet (refer to Fig. 1) 106 is fixed to the upper face of the upper cover 76 and the magnet 106 sticks to the housing of the placement section due to the upper cover 76 being lifted. By sticking the magnet 106 to the housing of the placement section in this manner, a state in which the upper cover 76 is lifted, that is, a state in which the cover housing 22 is exposed is maintained.

The stage 26 is substantially disc shaped and a petri-dish 110 is placed on the upper face of the stage 26. The outer diameter of the stage 26 is smaller than the outer diameter of the lower cover 78. The stage 26 is installed on the upper face of the lower cover 78.

As illustrated in Fig. 7, the lifting and lowering device 28 includes a support rod 112, a rack 114, a pinion 116, and an electromagnetic motor (refer to Fig. 10) 117. A through hole (not illustrated) which penetrates the lower cover 78 in the up-down direction is formed in the lower cover 78 and the support rod 112 is inserted through the through hole. The outer diameter of the support rod 112 is smaller than the inner diameter of the through hole and the support rod 112 is capable of moving in the up-down direction, that is, the Z-direction. The lower face of the stage 26 is fixed to the upper end of the support rod 112.

The rack 114 is fixed to the outer circumferential surface of a portion of the support rod 112 which extends downward from the lower cover 78 such that the rack 114 extends in the axial direction of the support rod 112. The pinion 116 meshes with the rack 114 and rotates through the driving of the electromagnetic motor 117. The pinion 116 is held by the housing of the placement section to be capable of rotating. According to this structure, the support rod 112 moves in the Z-direction and the stage 26 is lifted and lowered by due to the pinion 116 rotating through the driving of the electromagnetic motor 117. A measurement rod 118 is provided to stand next to the stage 26 on the upper face of the lower cover 78. A scale is denoted on the outer circumferential surface of the measurement rod 118 and it is possible to check the height in the Z-direction of the stage 26, that is, the lifting and lowering amount of the stage 26 by visual inspection using the scale.

As illustrated in Fig. 9, the cooling device 30 includes a cooling water path 120, a circulation device 122, and a pipe 124. The cooling water path 120 penetrates the inner portion of the stage 26 along the surface direction in a "]" shape and is open at two locations on the outer circumferential surface of the stage 26. The circulation device 122 is a device which cools water and circulates the cooled water. The circulation device 122 is connected to the cooling water path 120 via the pipe 124. Accordingly, the stage 26 is cooled due to the cooling water flowing in the inner portion of the stage 26.

As illustrated in Fig. 5, the purge gas supplying mechanism 32 includes four air joints (in the diagram, three are illustrated) 130 and a purge gas supply device (refer to Fig. 10) 132. The four air joints 130 are provided at equidistant positions on the upper end portion of the side face of the upper cover 76 and each of the air joints 130 is open to the inner portion of the upper cover 76. The purge gas supply device 132 is a device which supplies an inert gas such as nitrogen and is connected to the air joints 130 via a pipe (not illustrated). According to this structure, the purge gas supplying mechanism 32 supplies an inert gas to the inner portion of the upper cover 76.

The concentration detection mechanism 34 includes an air joint 140, a pipe 142, and a detection sensor (refer to Fig. 10) 144. A through hole (not illustrated) connects the upper face to the side face of the lower cover 78 is formed in the lower cover 78. An opening 146 of the upper face side of the lower cover 78 of the through hole is positioned on the inside of the packing 82. Meanwhile, the air joint 140 is connected to the opening of the side face side of the lower cover 78 of the through hole. The detection sensor 144 is a sensor which detects an oxygen concentration and is connected to the air joint 140 via the pipe 142. According to this structure, the concentration detection mechanism 34 detects the oxygen concentration in the inner portion of the cover housing 22 when the cover housing 22 is sealed.

As illustrated in Fig. 1, the ventilation mechanism 36 includes an L-shaped pipe 150, a connecting pipe 152, and a main pipe 154. As illustrated in Fig. 7, a duct port 160 which is open to the upper face and the lower face of the lower cover 78 is formed in the lower cover 78. The opening of the upper face side of the lower cover 78 of the duct port 160 is a tapered surface 162 in which the inner diameter increases in size toward the top. In other words, when the cover housing 22 is sealed, the tapered surface 162 assumes a state of being inclined toward the inner wall surface of the upper cover 76. Meanwhile, the L-shaped pipe 150 is connected to the opening of the lower face side of the lower cover 78 of the duct port 160. The main pipe 154 is connected the L-shaped pipe 150 via the connecting pipe 152. The portion of the connecting pipe 152 on the L-shaped pipe 150 is omitted. An ozone filter 166 is installed on the inner portion of the main pipe 154. The ozone filter 166 is formed of activated carbon and adsorbs ozone.

As illustrated in Fig. 10, the control device 38 is provided with a controller 170 and multiple drive circuits 172 . The multiple drive circuits 172 are connected to the electrodes 56, the process gas supply device 74, the electromagnetic motor 117, the circulation device 122 and the purge gas supply device 132. The controller 170 is provided with a CPU, a ROM, a RAM, and the like, the main constituent of the controller 170 is a computer, and the controller 170 is connected to the multiple drive circuits 172. Accordingly, the operations of the plasma generation device 20, the lifting and lowering device 28, the cooling device 30, and the purge gas supplying mechanism 32 are controlled by the controller 170. The controller 170 is connected to the detection sensor 144. Accordingly, the controller 170 acquires the detection result of the detection sensor 144, that is, the oxygen concentration of the inner portion of the cover housing 22.

### <Plasma Irradiation by Atmospheric Pressure Plasma Irradiation Apparatus>

Since the culture medium is activated by irradiating the culture medium with the plasma, utilization of the plasma is anticipated in the field of medicine such as in the treatment of cancer in which a plasma-irradiated culture medium is used. Therefore, experiments using a plasma-irradiated culture medium and the like are performed and it is preferable that a culture medium which is used in an experiment be irradiated with plasma in a state in which the conditions during plasma irradiation are managed. According to the configuration which is described above, in the atmospheric pressure plasma irradiation apparatus 10, by placing the petri-dish 110 in which the culture medium is stored on the stage 26 sealing the cover housing 22, it is possible to irradiate the culture medium with the plasma under predetermined conditions . Hereinafter, a detailed description will be given of a method of irradiating the culture medium with the plasma under predetermined conditions.

Specifically, first, the petri-dish 110 in which the culture medium is stored is placed on the stage 26. Next, the stage 26 is lifted or lowered to an arbitrary height by the lifting and lowering device 28. Accordingly, it becomes possible to arbitrarily set the distance between the ejection port 72 of the plasma and the culture medium which serves as the irradiation target object of the plasma. It is possible to check the lifting or lowering height of the stage 26 using the scale of the measurement rod 118.

Next, the upper cover 76 is lowered and the cover housing 22 is sealed. An inert gas is supplied to the inner portion of the cover housing 22 by the purge gas supplying mechanism 32. At this time, the oxygen concentration inside the cover housing 22 is detected by the concentration detection mechanism 34. After the detected oxygen concentration becomes less than or equal to a preset threshold, the plasma is ejected by the plasma generation device 20 into the inner portion of the cover housing 22. During the irradiation of the plasma, the supply of the inert gas to the inner portion of the cover housing 22 is performed continuously.

In this manner, due to the inert gas being supplied to the inner portion of the cover housing 22, the air inside the cover housing 22 is vented to the outside of the cover housing 22. At this time, the conditions which influence the plasma irradiation are managed by adjusting the oxygen concentration inside the cover housing 22. In detail, since the plasma contains active radicals, when the active radicals react with oxygen, the active radicals become ozone and the effect of the plasma irradiation is reduced. Therefore, by adjusting the oxygen concentration inside the cover housing 22, it is possible to investigate the influence of the oxygen concentration on the effect of the plasma-irradiated culture medium. It becomes possible to irradiate the culture medium with the plasma under the same conditions. Accordingly, it becomes possible to effectively perform experiments.

As described above, in the atmospheric pressure plasma irradiation apparatus 10, the distance between the ejection port 72 of the plasma and the culture medium is arbitrarily set. Accordingly, it becomes possible to investigate the influence of the irradiation distance on the effect of the plasma-irradiated culture medium and it becomes possible to effectively perform experiments.

During the plasma irradiation, cooling water is caused to circulate in the inner portion of the stage 26 by the cooling device 30. Accordingly, due to the stage 26 being cooled, it becomes possible to reduce an increase in the temperature of the culture medium inside the petri-dish 110 caused by plasma irradiation and to prevent the evaporation of the culture medium. By managing the temperature of the cooling water, it becomes possible to adjust the temperature of the culture medium during the plasma irradiation.

The duct port 160 is formed in the lower cover 78. Therefore, the inside of the cover housing 22 assumes a positive pressure through the supplying of an inert gas to the inside of the cover housing 22 and ventilation from the inside of the cover housing 22 occurs naturally. The tapered surface 162 in which the inner diameter increases in size toward the upper face of the lower cover 78 is formed on the duct port 160 of the lower cover 78. Accordingly, it is possible to promote the ventilation of gas from the inner portion of the cover housing 22. The ozone filter 166 is provided in the ventilation mechanism 36. Accordingly, even in a case in which the plasma reacts with the oxygen and ozone is generated, it becomes possible to prevent the ventilation of ozone to the outside.

Since the glass plate 80 through which it is possible to visually recognize the inner portion of the upper cover 76 is installed in the upper cover 76, it is possible to check the status of the plasma irradiation.

Incidentally, in the example, the atmospheric pressure plasma irradiation apparatus 10 is an example of a plasma irradiation apparatus. The plasma generation device 20 is an example of a plasma generation device. The cover housing 22 is an example of a cover housing. The stage 26 is an example of a stage. The lifting and lowering device 28 is an example of a moving device. The cooling device 30 is an example of a cooling device. The control device 38 is an example of a control device. The ejection port 72 is an example of an ejection port. The purge gas supply device 132 is an example of a gas supply device. The detection sensor 144 is an example of a detection sensor. The duct port 160 is an example of a duct port. The tapered surface 162 is an example of a tapered surface.
The present invention is not limited to the example and it is possible to carry out the present invention in various modes subjected to various modifications are possible within the scope of the appended claims. Specifically, for example, in the example, the detection sensor 144 is a sensor for detecting oxygen concentration; however, it is possible to adopt a sensor for detecting another specific gas.

The present invention is not limited to the field of medicine and it is possible to apply the present invention to various fields such as the engineering field.

### Reference Signs List

10: atmospheric pressure plasma irradiation apparatus, 20: plasma generation device, 22: cover housing, 26: stage, 28: lifting and lowering device (moving device), 30: cooling device, 38: control device, 72: ejection port, 132: purge gas supply device (gas supply device), 144: detection sensor, 160:

## Claims

1. A plasma irradiation apparatus (10) comprising:
a cover housing (22) which is configured to form a predetermined space by partitioning;
a plasma generation device (20) which is configured to eject the plasma toward an inner portion of the cover housing (22);
a purge gas supply device (132) which is configured to supply a gas to the inner portion of the cover housing (22);
a stage (26) which is installed in the inner portion of the cover housing (22) and which is for placing a processing target liquid; and
a moving device (28) for moving at least one of an ejection port (72) of the plasma generation device (20) from which the plasma is ejected into the inner portion of the cover housing (22) and the stage (26) and arbitrarily changing a distance between the ejection port (72) and the stage (26),
the apparatus (10) is configured to irradiate the processing target liquid which is placed in the inner portion of the cover housing (22) with a plasma; **characterized by**
a control device (38) which is configured to control operations of the plasma generation device (20) and the purge gas supply device (132),
wherein the control device (38) is configured to control operations of the plasma generation device (20) and the purge gas supply device (132) such that a gas is supplied to the inner portion of the cover housing (22) using the purge gas supply device (132), the plasma is subsequently ejected toward the inner portion of the cover housing (22) using the plasma generation device (20), and when the plasma is being ejected, the gas is supplied to the inner portion of the cover housing (22) using the purge gas supply device (132),
wherein the plasma irradiation apparatus (10) comprises further
a detection sensor (144) which is configured to detect a concentration of a predetermined gas in the inner portion of the cover housing (22); and
a cooling device (30) which is configured to cool the stage (26).

2. The plasma irradiation apparatus (10) according to Claim 1,
wherein the cover housing (22) includes a duct port (160) for venting a gas from the inner portion of the cover housing (22), and
wherein a tapered surface (162) which is inclined toward an inner wall surface of the cover housing (22) is formed on an opening section of the duct port (160) to the inner portion of the cover housing (22).

## Patentansprüche

1. Plasmabestrahlungsvorrichtung (10), umfassend:
ein Abdeckgehäuse (22), das so ausgeführt ist, dass es durch Unterteilung einen vorgegebenen Raum bildet;
eine Plasmaerzeugungs-Einrichtung (20), die so ausgeführt ist, dass sie das Plasma in Richtung eines inneren Abschnitts des Abdeckgehäuses (22) ausstößt;
eine Einrichtung (132) für Zufuhr von Spülgas, die so ausgeführt ist, dass sie dem inneren Abschnitt des Abdeckgehäuses (22) ein Gas zuführt;
einen Tisch (26), der in dem inneren Abschnitt des Abdeckgehäuses (22) installiert ist und zum Aufbringen von Verarbeitungsobjekt-Flüssigkeit dient; sowie
eine Bewegungs-Einrichtung (28) zum Bewegen einer Ausstoßöffnung (72) der Plasmaerzeugungs-Einrichtung (20), über die das Plasma in den inneren Abschnitt des Abdeckgehäuses (22) hinein ausgestoßen wird, oder/und des Tischs (26) sowie zum beliebigen Ändern eines Abstandes zwischen der Ausstoßöffnung (72) und dem Tisch (26),
wobei die Vorrichtung (10) so ausgeführt ist, dass sie die Verarbeitungsobjekt-Flüssigkeit, die sich in dem inneren Abschnitt des Abdeckgehäuses (22) aufgebracht ist, mit einem Plasma bestrahlt; **gekennzeichnet durch**:
eine Steuerungs-Einrichtung (38), die so ausgeführt ist, dass sie Funktionen der Plasmaerzeugungs-Einrichtung (20) und der Einrichtung (132) für Zufuhr von Spülgas steuert,
wobei die Steuerungs-Einrichtung (38) so ausgeführt ist, dass sie Funktionen der Plasmaerzeugungs-Einrichtung (20) und der Einrichtung (132) für Zufuhr von Spülgas so steuert, dass dem inneren Abschnitt des Abdeckgehäuses (22) unter Verwendung der Einrichtung (132) für Zufuhr von Spülgas ein Gas zugeführt wird, so steuert, dass in Betrieb das Plasma anschließend unter Verwendung der Plasmaerzeugungs-Einrichtung (20) in Richtung des inneren Abschnitts des Abdeckgehäuses (22) ausgestoßen wird, und so steuert, dass in Betrieb, wenn das Plasma ausgestoßen wird, das Gas dem inneren Abschnitt des Abdeckgehäuses (22) unter Verwendung der Einrichtung (132) für Zufuhr von Spülgas zugeführt wird,
wobei die Plasmabestrahlungsvorrichtung (10) des Weiteren umfasst:
einen Erfassungssensor (144), der so ausgeführt ist, dass er eine Konzentration eines vorgegebenen Gases in dem inneren Abschnitt des Abdeckgehäuses (22) erfasst; und
eine Kühlvorrichtung (30), die zum Kühlen des Tischs (26) ausgeführt ist.

2. Plasmabestrahlungsvorrichtung (10) nach Anspruch 1,
wobei das Abdeckgehäuse (22) eine Kanalöffnung (160) zum Ablassen eines Gases aus dem inneren Abschnitt des Abdeckgehäuses (22) enthält, und
wobei eine abgeschrägte Fläche (162), die zu einer Innenwandfläche des Abdeckgehäuses (22) hin geneigt ist, an einem Öffnungsabschnitt der Kanalöffnung (160) zu dem inneren Abschnitt des Abdeckgehäuses (22) ausgebildet ist.

## Revendications

1. Appareil d'irradiation plasma (10) comprenant :
une enveloppe de couverture (22) qui est configurée pour former un espace prédéterminé par séparation,
un dispositif de génération de plasma (20) qui est configuré pour délivrer un gaz à la partie interne de l'enveloppe de couverture (22),
un dispositif de fourniture de gaz de purge (132) qui est configuré pour délivrer un gaz à la partie interne de l'enveloppe de couverture (22),
un étage (26) qui est installé dans la partie interne de l'enveloppe de couverture (22) et qui est destiné à placer un liquide cible de traitement, et
un dispositif de déplacement (28) destiné à déplacer au moins l'un d'un orifice d'éjection (72) du dispositif de génération de plasma (20) à partir duquel est éjecté le plasma dans la partie interne de l'enveloppe de couverture (22), et destiné à modifier arbitrairement la distance entre l'orifice d'éjection (72) et l'étage (26),
l'appareil (10) est configuré pour irradier avec un plasma le liquide cible de traitement qui est placé dans la partie interne de l'enveloppe de couverture (22), **caractérisé par** :
un dispositif de commande (38) qui est configuré pour commander les opérations du dispositif de génération de plasma (20) et du dispositif de fourniture de gaz de purge (132),
dans lequel le dispositif de commande (38) est configuré pour commander les opérations du dispositif de génération de plasma (20) et du dispositif de fourniture de gaz de purge (132) de sorte à ce qu'un gaz soit délivré à la partie interne de l'enveloppe de couverture (22) en utilisant le dispositif de fourniture de gaz de purge (132), et de sorte à ce qu'en service le plasma soit ensuite éjecté vers la partie interne de l'enveloppe de couverture (22) en utilisant le dispositif de génération de plasma (20), et de sorte que, en service, lorsque le plasma est éjecté, le gaz est délivré à la partie interne de l'enveloppe de couverture (22) en utilisant le dispositif de fourniture de gaz de purge (132),
où l'appareil d'irradiation plasma (10) comprend en outre :
un capteur de détection (144) qui est configuré pour détecter la concentration d'un gaz prédéterminé dans la partie interne de l'enveloppe de couverture (22), et
un dispositif de refroidissement (30) qui est configuré pour refroidir l'étage (26).

2. Appareil irradiation plasma (10) selon la revendication 1,
dans lequel l'enveloppe de couverture (22) inclut l'orifice d'un conduit (160) destiné à évacuer un gaz de la partie interne de l'enveloppe de couverture (22), et
dans lequel une surface conique (162), qui est inclinée vers une surface de paroi interne de l'enveloppe de couverture (22), est formée sur une section ouvrante de l'orifice de conduit (160) vers la partie interne de l'enveloppe de couverture (22).
